(19) Europäisches Patentamt
European Patent Office
Office européen des brevets

(11) **EP 2 796 185 A1**

## (12) EUROPEAN PATENT APPLICATION
published in accordance with Art. 153(4) EPC

(43) Date of publication:
**29.10.2014 Bulletin 2014/44**

(21) Application number: **12861033.4**

(22) Date of filing: **14.12.2012**

(51) Int Cl.:
**B01D 63/02** (2006.01)    **A61M 1/18** (2006.01)

(86) International application number:
**PCT/JP2012/082510**

(87) International publication number:
**WO 2013/094533 (27.06.2013 Gazette 2013/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2011   JP 2011276761**

(71) Applicant: **Toray Industries, Inc.**
**Tokyo, 103-8666 (JP)**

(72) Inventors:
• **NAKANO, Yoshinori**
  **Okazaki-shi**
  **Aichi 444-8522 (JP)**
• **NAKAMATSU, Osamu**
  **Okazaki-shi**
  **Aichi 444-8522 (JP)**

(74) Representative: **Prüfer & Partner GbR**
**European Patent Attorneys**
**Sohnckestrasse 12**
**81479 München (DE)**

## (54) HOLLOW FIBER MEMBRANE MODULE AND CASING TUBE USED FOR SAME

(57)    Provided is a hollow-fiber membrane module including: a cylindrical casing having a treating-liquid inlet port and a treating-liquid outlet port; a hollow-fiber membrane bundle mounted within the cylindrical casing while being aligned in one direction; potting layers fixing both ends of the hollow-fiber membrane bundle to an inner wall of the cylindrical casing such that hollow sections of hollow-fiber membranes are open; and header members surrounding opened end surfaces of the hollow-fiber membrane bundle, the header members being attached to respective ends of the cylindrical casing and having a treated-liquid inlet port and a treated-liquid outlet port, wherein the hollow-fiber membrane module further includes an upper baffle plate provided in the cylindrical casing at a position immediately below the treating-liquid inlet port with a predetermined gap from the inner opening of the treating-liquid inlet port of the cylindrical casing, and a lower baffle plate provided at a position at which the lower baffle plate is line symmetric to the upper baffle plate with respect to an axis of the casing, and no baffle plate is present on an inner circumference of the cylindrical casing between the upper baffle plate and the lower baffle plate. Consequently, the flow speed of the treating liquid can be made more uniform, and the hollow-fiber membrane module can be adopted industrially.

Fig. 1

**Description**

TECHNICAL FIELD

The present invention relates to a hollow-fiber membrane module used in artificial dialysis, blood treatment devices necessary for hemofiltration, water purifiers, gas separation films, and the like, and a cylindrical casing used for the hollow-fiber membrane.

BACKGROUND ART

The hollow-fiber membrane module that filters and dialyzes substances by using treating liquid and treated liquid has an effective membrane area per unit volume and therefore, has been used in many fields including water treatment such as precision filtration and ultrafiltration, gas separation of nitrogen, oxygen, hydrogen, and the like, drugs, and biotechnology, and has been suitably used especially as a blood treatment device for hemodialysis and hemofiltration (hereinafter referred to as dialyzer). The dialyzer will be mainly described below in detail.

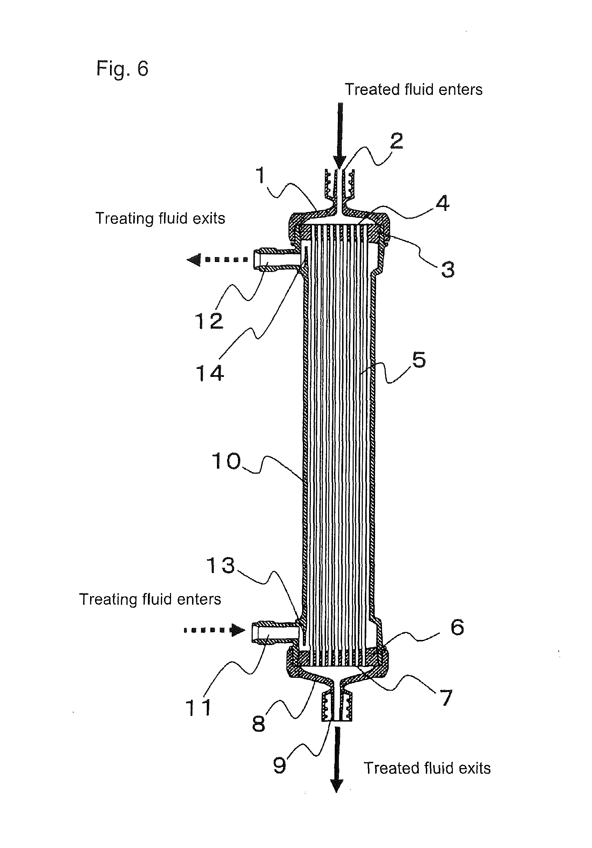

For example, as shown in Fig. 6, the dialyzer includes a cylindrical casing 10 having a treating-liquid inlet port 11 at one end and a treating-liquid outlet port 12 at the other end, a hollow-fiber membrane bundle 5 mounted in the cylindrical casing 10, potting layers 3 and 6 that fix the hollow-fiber membrane bundle 5 to an inner wall of the cylindrical casing 10 at the respective ends, and header members 1 and 8 attached to both ends of the cylindrical casing 10 so as to surround entire opened surfaces 4 and 7 of hollow sections of the hollow-fiber membrane bundle 5, which are formed outside the surfaces of the potting layers 3 and 6. The header members 1 and 8 are provided with a treated-liquid inlet port 2 and a treated-liquid outlet port 9, respectively. In the case where the hollow-fiber membrane module is used as the dialyzer, the treated liquid is blood, and the treating liquid is dialysis fluid.

In such a dialyzer, for example, by causing blood to flow on the inner sides of the hollow-fiber membranes and dialysis fluid to flow on the outer sides of the hollow-fiber membranes to generate filtration due to pressure difference and diffusion due to concentration difference, substances to be removed, such as urotoxin and $\beta$2-microglobulin in blood, can be moved toward the dialysis fluid side through the hollow-fiber membranes to purify blood. That is, blood entering from the treated-liquid inlet port 2 of the header member 1 flows into the hollow-fiber membranes through an opened surface 4 of a hollow section of the potting layer 3, further flows on the inner sides of the hollow-fiber membranes, passes through an opened surface 7 of a hollow section of the potting layer 6 at the other end, and finally exits from the treated-liquid outlet port 9 provided at the header member 8. Dialysis fluid entering from the treating-liquid inlet port 11 of the cylindrical casing 10 flows on the outer sides of the hollow-fiber membranes, and exits from the treating-liquid outlet port 12. As shown in Fig. 6, generally, the treated liquid and the treating liquid are caused to flow in opposed directions in a so-called counter-flow manner. This can achieve satisfactory substance exchange through the hollow-fiber membranes.

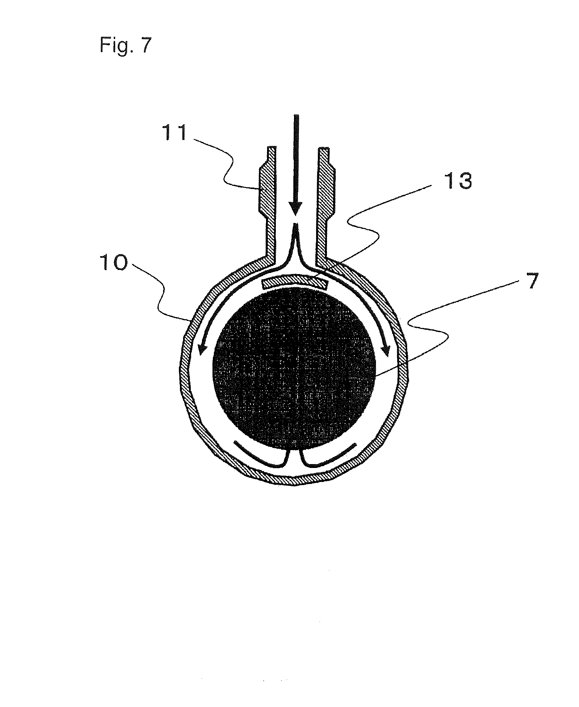

To satisfactorily achieve such substance exchange through the hollow-fiber membranes in the dialyzer, it is effective to cause the dialysis fluid to uniformly flow on the outer sides of each of the hollow-fiber membranes. When the flow of the dialysis fluid is imbalanced, the purifying performance of the blood purifier is lowered as a whole. In the conventional dialyzer, the dialysis-fluid flow rate on the side opposed to the dialysis fluid inlet in the cross section of the cylindrical casing increases, thereby generating irregularity of the flow of the dialysis fluid. This is because, as shown in Fig. 7, the dialysis fluid collides against an upper baffle plate 13 located immediately below the treating-liquid inlet port 11, is dispersed, and merges again under the guide of the inner circumference of the cylindrical casing to increase the flow rate.

Many patent applications have proposed improvements for making the dialysis-fluid flow more uniform by devising the shape of the cylindrical casing. For example, Patent Document 1 discloses the technique of improving the treating-liquid flow by closing the section between one side of the baffle plate provided at the treating-liquid inlet and the inner circumferential surface of the casing. However, the treating liquid colliding against the baffle plate located immediately below the port is guided by the inner circumference of the cylindrical casing in one direction, circulates on the inner circumferential surface of the cross section of the casing and finally, collides against the wall of the baffle plate to locally make the flow rate of the treating liquid nonuniform.

Patent Document 2 describes the technique of disposing a plurality of baffle plates on the whole circumference near the end of the cylindrical casing, and providing, between the adjacent baffle plates, a slit extending in an oblique direction with respect to the axis of the cylindrical casing. However, there is a problem that part of the baffle plate disadvantageously blocks the treating-liquid flow.

PRIOR ART DOCUMENTS

PATENT DOCUMENTS

Patent Document 1: Japanese Patent Laid-open Publication No. 2005-296265
Patent Document 2: Japanese Patent Laid-open Publication No. 2004-154772

SUMMARY OF THE INVENTION

PROBLEMS TO BE SOLVED BY THE INVENTION

In view of the above problem of the conventional art, an object of the present invention is to provide a hollow-fiber membrane module and a cylindrical casing used in the hollow-fiber membrane module which can make the flow rate of the treating liquid more uniform and can be adopted industrially.

SOLUTIONS TO THE PROBLEMS

To achieve the above object, the present invention is characterized by any of following configurations.

1. A hollow-fiber membrane module including: a cylindrical casing having a treating-liquid inlet port and a treating-liquid outlet port; a hollow-fiber membrane bundle mounted within the cylindrical casing while being aligned in one direction; potting layers fixing both ends of the hollow-fiber membrane bundle to an inner wall of the cylindrical casing such that hollow sections of hollow-fiber membranes are open; and header members surrounding opened end surfaces of the hollow-fiber membrane bundle, the header members attached to respective ends of the cylindrical casing and having a treated-liquid inlet port and a treated-liquid outlet port, wherein the hollow-fiber membrane module further includes an upper baffle plate provided in the cylindrical casing at a position immediately below the treating-liquid inlet port with a predetermined gap from an inner opening of the treating-liquid inlet port of the cylindrical casing, and a lower baffle plate provided at a position at which the lower baffle plate is line symmetric to the upper baffle plate with respect to an axis of the casing, and no baffle plate is present on an inner circumference of the cylindrical casing between the upper baffle plate and the lower baffle plate.

2. A cylindrical casing used in a hollow-fiber membrane module, the casing comprising a treating-liquid inlet port and a treating-liquid outlet port at a side of a body, wherein the casing further includes an upper baffle plate provided in the cylindrical casing at a position immediately below the treating-liquid inlet port with a predetermined gap from an inner opening of the treating-liquid inlet port of the cylindrical casing, and a lower baffle plate provided at a position at which the lower baffle plate is line symmetric to the upper baffle plate with respect to an axis of the casing, the upper baffle plate covers the inner opening of the port when the port is viewed from below, and no baffle plate is present on an inner circumference of the cylindrical casing between the upper baffle plate and the lower baffle plate.

The upper baffle plate is preferably is provided at the same position as the position of the upper baffle plate in the conventional dialyzer in Fig. 7, which is described in the section of Background Art. One of main characteristics of the present invention is that the lower baffle plate is provided at a position at which the lower baffle plate is line symmetric to the upper baffle plate with respect to the axis of the casing.

As described later in detail, the position at which the lower baffle plate is line symmetric to the upper baffle plate with respect to the axis of the casing is not limited to the case where the upper baffle plate is accurately line symmetric to the lower baffle plate, and the shape and size of the lower baffle plate are not necessarily the same as those of the upper baffle plate.

The predetermined gap is not limited to be within a certain range, and can vary depending on the size of the module, and a gap S defined below may be dimensioned such that the baffle plate does not completely close the inner opening of the treating-liquid port. Since the lower baffle plate is line symmetric to the upper baffle plate with respect to the axis of the casing, a below-mentioned gap S' between the lower baffle and the inner circumferential surface of the cylindrical casing preferably has the same distance as the gap S. Accordingly, in the typical dialyzer, S and S' is preferably 2 mm or more, and more preferably 3 mm or more. Moreover, S and S' is preferably 6 mm or less, and more preferably 5 mm or less.

On the other hand, the upper baffle plate preferably covers the inner opening of the treating-liquid inlet port when the port is viewed from below, such that the treating liquid collides against the baffle and flows in the inner circumferential direction of the cylindrical casing.

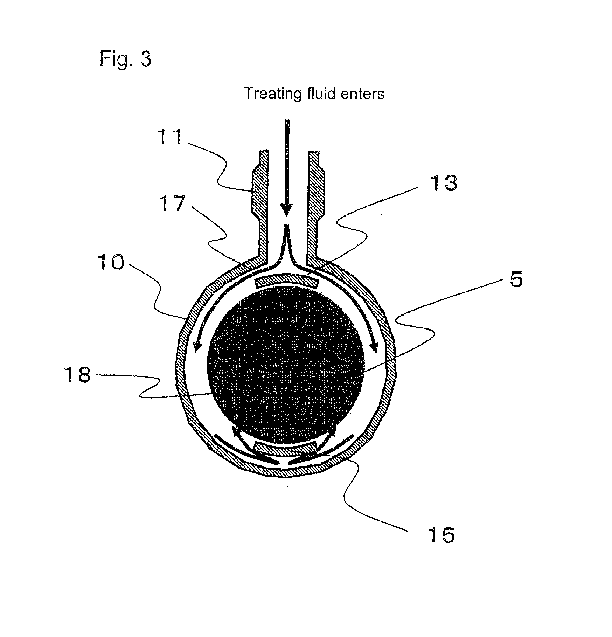

As described above, according to the present invention, no baffle plate is present on the inner circumference of the cylindrical casing between the upper baffle plate and the lower baffle plate. That is, as shown in an aspect in Fig. 3, no baffle plate is present in the cross section immediately below the inlet of the treating-liquid port, except for one upper baffle plate and one lower baffle plate.

Preferably, the lower baffle plates and the upper baffle plates are disposed below both of the treating-liquid inlet port and the treating-liquid outlet port.

Given that an inner diameter of the treating-liquid inlet port is di, and a longitudinal length of the upper baffle plate and a longitudinal length of the lower baffle plate below the treating-liquid inlet port when viewed from the cross section of the module are Wi1 and Wi2, respectively, it is preferred that $di \leq Wi1 \leq 3di$ and $di \leq Wi2 \leq 3di$ hold.

Similarly, given that an inner diameter of the treating-liquid outlet port is do, and a length of the upper baffle plate and a length of the lower baffle plate below the treating-liquid outlet port, which correspond to Wi1 and Wi2, are Wo1 and Wo2, respectively, it is preferred that $do \leq Wo1 \leq 3do$ and $do \leq Wo2 \leq 3do$ hold.

EFFECTS OF THE INVENTION

According to the present invention, the upper baffle plate is disposed immediately below the treating-liquid port in the cylindrical casing with a predetermined gap from the inner opening of the treating-liquid inlet port, and the lower baffle plate is line symmetric to the upper baffle plate with respect to the axis of the casing. Thus, when treating liquid is introduced from the treating-liquid inlet port, the treating liquid is dispersed in the circumferential direction by the upper baffle plate located immediately below the treating-liquid inlet port, is guided to the inner circumference of the cylindrical casing and then, merges at a position opposed to the treating-liquid inlet port. However, the treating liquid collides against the lower baffle plate to be diffused to the surroundings, thereby preventing an increase in the flow rate. As a result, flows of the treating-liquid become more uniform. The flow of the treating-liquid in the hollow-fiber membrane module becomes uniform, thereby improving the performance of the module.

BRIEF DESCRIPTION OF THE DRAWINGS

Fig. 1 is a schematic vertical sectional view of a hollow-fiber membrane module according to an embodiment of the present invention.

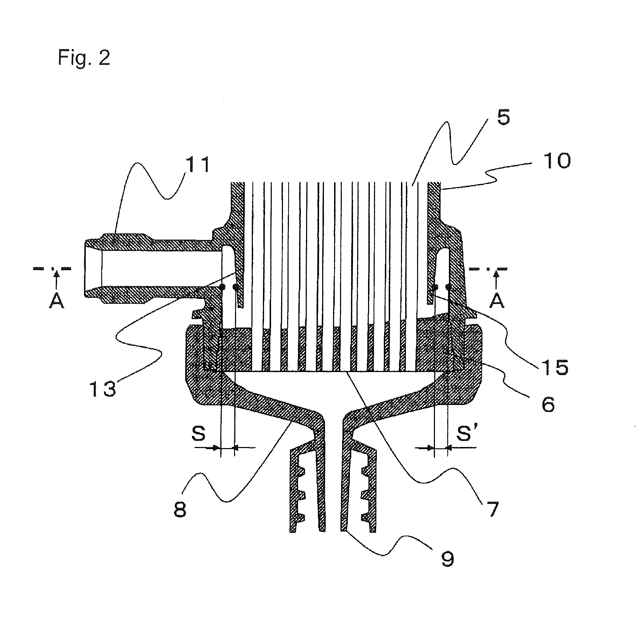

Fig. 2 is a schematic partial vertical sectional view showing an end of the hollow-fiber membrane module in Fig. 1 on the side of a dialysis-fluid inlet port.

Fig. 3 is a schematic partial sectional view taken along A-A showing an end of the hollow-fiber membrane module in Fig. 2 on the side of a treating-liquid inlet port.

Fig. 4 is a schematic partial sectional view showing an end of the hollow-fiber membrane module in Fig. 1 on the side of the treating-liquid inlet port and the treating-liquid outlet port, which is cut along the circumference of the cylindrical casing with the hollow-fiber membrane bundle 5 being removed.

Fig. 5 is a schematic partial vertical sectional view showing an end of the cylindrical casing of the hollow-fiber membrane module in Fig. 1 with the fiber bundle 5, a potting layer 6, and a header 8 being removed.

Fig. 6 is a schematic vertical sectional view of a conventional hollow-fiber membrane module.

Fig. 7 is a schematic partial sectional view showing an end of the hollow-fiber membrane module in Fig. 6 on the side of the treating-liquid inlet port when cut along the circumference of the cylindrical casing.

EMBODIMENTS OF THE INVENTION

First, a hollow-fiber membrane module according to the present invention will be described with reference to the drawings.

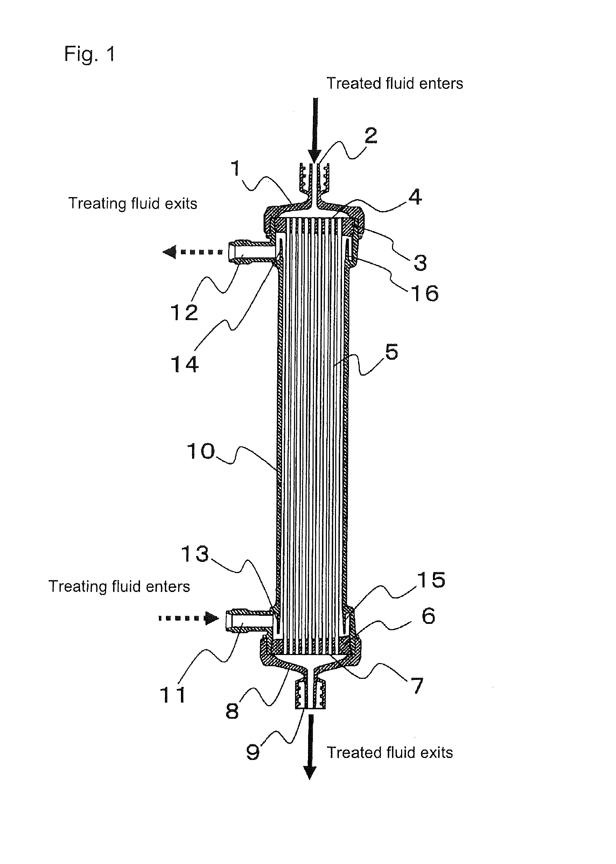

Fig. 1 shows an example of a hollow-fiber membrane module according to the present invention. In Fig. 1, the hollow-fiber membrane module is configured by a cylindrical casing 10, a hollow-fiber membrane bundle 5 mounted in the cylindrical casing 10, potting layers 3 and 6 fixing both ends of a hollow-fiber membrane bundle 5 to an inner wall of the cylindrical casing 10 such that hollow sections of hollow-fiber membranes are opened, a header member 1 attached to one end of the cylindrical casing 10, and a header member 8 attached to the other end of the cylindrical casing 10.

The cylindrical casing 10 is a cylindrical member in which the hollow-fiber membrane bundle 5 is mounted, and includes a treating-liquid inlet port 11 at a side of its body near one end and a treating-liquid outlet port 12 at the side of the body near the other end. The material for the casing is not particularly limited, and a cylindrical casing made of plastic such as polypropylene, polyethylene, polytetrafluoroethylene, polyester, polycarbonate, or ABS (acrylonitrile-butadiene-styrene) is suitably used.

The hollow-fiber membrane bundle 5 is formed of a plurality of hollow-fiber membranes (semipermeable membranes) aligned in one direction (axial direction of the cylindrical casing 10) and is mounted in the cylindrical casing 10. Ends of the hollow-fiber membrane bundle 5 are fixed to the inner wall of the cylindrical casing 10 such that the hollow sections are opened by the potting layers 3 and 6. The hollow-fiber membrane is not particularly limited, but a hollow-fiber membrane made of a material such as acrylic, polysulfone, polyethersulfone, cellulose, triacetate, polyethylene, and polypropylene is suitably used.

The potting layers 3 and 6 fixes the hollow-fiber membrane bundle at both ends of the cylindrical casing, and in the case of using the hollow-fiber membrane bundle as the hollow-fiber membrane module, the potting layers 3 and 6 separate a region where the treated liquid flows from a region where the treating liquid flows. Basically, the potting layers are formed by casting a polyurethane, silicone, or epoxy high-polymer material (in other words, two-liquid mixed hardening-type high-polymer adhesive).

The header member 1 is attached to one end of the cylindrical casing, and the header member 8 is attached to the other end of the cylindrical casing. The header member 1 includes a treated-liquid inlet port 2, and is attached to one end of the cylindrical casing 10 so as to surround an opened end surface 4 of a hollow section of the hollow-fiber membranes. The header member 8 includes a treated-liquid outlet port 9, and is attached to the other end of the cylindrical casing 10 so as to surround an opened end surface 7 of a hollow section of the hollow-fiber membranes. The header members 1 and 8 are preferably attached to the both ends of the cylindrical casing 10 in a liquid-tight manner. The header members 1 and 8 may be made of the same material as the material for the cylindrical casing 10.

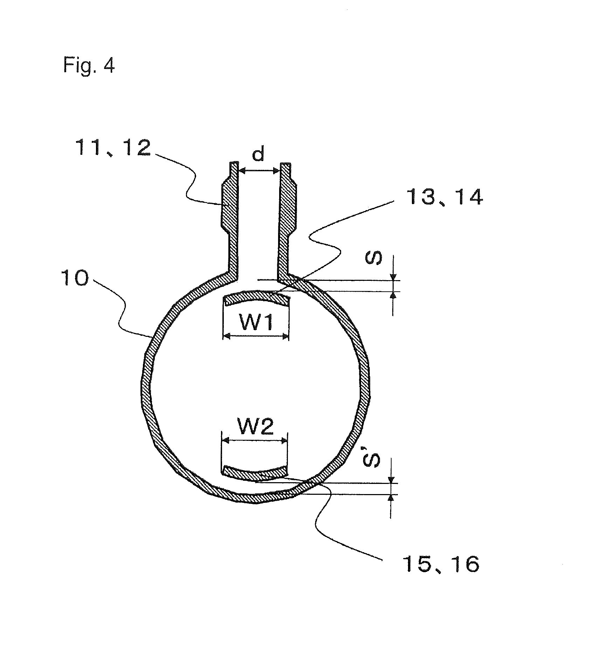

In the hollow-fiber membrane module shown in Fig. 1, an upper baffle plate 13 is provided immediately below the treating-liquid inlet port 11, and an upper baffle plate 14 is provided immediately below the treating-liquid outlet port 12. Further, in the hollow-fiber membrane module according to the present invention, a lower baffle plate 15 is provided at a position where the lower baffle plate 15 is line symmetric to the upper baffle plate 13 located immediately below the treating-liquid inlet port with respect to the axis of the casing. In a preferred embodiment shown in Fig. 1, a lower baffle plate 16 is provided at a position where the lower baffle plate 16 is line symmetric to the upper baffle plate 14 located immediately below the treating-liquid outlet port with respect to the axis of the casing. The axis of the casing is an axis passing the center of a cross section perpendicular to the direction in which the hollow-fiber membrane bundle is aligned in the cylindrical casing, and that the upper baffle plate is line symmetric to the lower baffle plate with respect to the axis of the casing is not limited to the case where the upper baffle plate is accurately line symmetric to the lower baffle plate with respect to the axis of the casing. One baffle plate only needs to be accurately line symmetric to the other baffle plate by 70% or more, and preferably, 80% or more, and more preferably, 90% or more. Both the plates may have different shapes and sizes. As shown in Fig. 4, the upper baffle plates 13 and 14 are provided with a predetermined gap S from an inner opening 17 of the treating-liquid port of the cylindrical casing. The gap S represents a minimum distance between a casing-side end of the inner opening of the treating-liquid port and the upper baffle plate. A gap S' in the lower baffle plate represents a minimum distance between the inner circumferential surface of the cylindrical casing and the lower baffle plate in the same position as the casing-side end of the inner opening of the treating-liquid port in the casing cross section. Both of the gaps may be appropriately determined depending on the type or size of the hollow-fiber membrane module. However, in the dialyzer, S and S' is preferably 2 mm or more, and more preferably 3 mm or more. Moreover, S and S' is preferably 6 mm or less, and more preferably 5 mm or less.

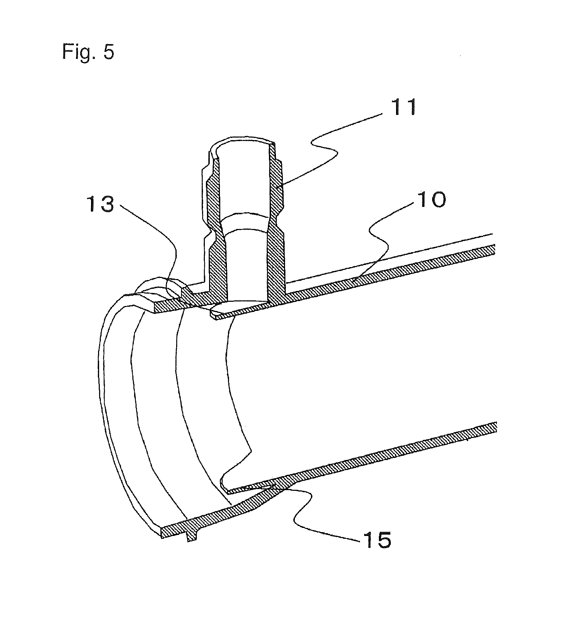

In Fig. 5, the treating-liquid port is disposed at a position near the end of the casing, the inner diameter of which is increased, and the upper and lower baffles extend from the bottom end of the diameter-increasing portion of the inner circumferential surface of the casing toward the front end of the casing. According to the present invention, there is no baffle plate present on the inner circumference of the cylindrical casing between the upper baffle plate and the lower baffle plate. Here, as shown as an inner circumference 18 in Fig. 3, the inner circumference of the cylindrical casing refers to the circumference in the cylindrical casing, in which the upper and lower baffles provided with the predetermined gap S from the inner circumferential surface are present, and preferably a range including its neighboring circumference. In the case where another baffle plate is present in the range, it is demonstrated from recent researches that the flow of the treating-liquid is blocked and thus becomes nonuniform. Preferably, the upper baffle plates 13 and 14 are dimensioned so as to cover the inner openings of the respective ports when viewed from below. Here, viewing the port from below means viewing the inner opening of the port from immediately below assuming that the front end side of the port is the upper side. In the case where it is difficult to view the port from below, for example, when the hollow-fiber membranes are inserted into the module, or when the casing is opaque, the port can be viewed from above to check whether or not the upper baffle plate covers the inner opening of the port. In any case, it is preferred that the treating liquid collides against the upper baffle plate and is dispersed in the inner circumferential direction of the cylindrical casing. Preferably, the front end of the upper baffle plates 13 and 14 and lower baffle plates 15 and 16 extends toward the potting layer. When viewed from the cross section of the module as shown in Fig. 4, it is given that a length of the upper baffle plate and a length of the lower baffle plate in a direction perpendicular to the axis of the treating-liquid port are W1 and W2, respectively, an inner diameter of the treating-liquid port is d, a length of the upper baffle plate and a length of the lower baffle plate below the treating-liquid inlet port are Wi1 and Wi2, respectively, to guide the flowing treating liquid to the inner circumferential surface of the cylindrical casing and make the treating-liquid flow more uniform, Wi1 and the inner diameter di of the treating-liquid inlet port on the casing inner circumferential surface preferably satisfy the relation: di ≤ Wi1 ≤ 3di. To cover variation in the treating-liquid merging position opposed to the position immediately below the treating-liquid port, which is caused by irregular flow rate in the treating-liquid inlet port, and make the treating-liquid flow more uniform, the width Wi2 of the lower baffle plates 15 and 16 and the inner diameter di preferably satisfy the relation: di ≤ Wi2 ≤ 3di. According to the present invention, when the upper baffle plate and the lower baffle plate are disposed below the treating-liquid outlet port as well as the treating-liquid inlet port, the cylindrical casing is symmetrical and therefore, the orientation of the casing need not be considered in the manufacturing process. Also in this case, however, when viewed from the cross section of the module, given that a length of the upper baffle plate and a length of the lower baffle plate in the direction perpendicular to the axis of the treating-liquid port are Wo1 and Wo2, respectively, and an inner diameter of the treating-liquid inlet port on the casing inner circumferential surface is do, it is preferred that the module is designed such that the relation: do ≤ Wo1 ≤ 3do and the relation: do ≤ Wo2 ≤ 3do hold. Note that W2 is not necessarily the same as W1.

Example

To produce the hollow-fiber membrane module for hemodialysis in Fig. 1, a cylindrical casing made of polycarbonate and a hollow-fiber membrane made of polysulfone disclosed in Example 1 of Japanese Patent Laid-open Publication No. 2001-170172 were prepared.

About 10000 hollow-fiber membranes were bundled and aligned in one direction to produce a hollow-fiber membrane bundle, and the hollow-fiber membrane bundle was inserted into the cylindrical casing in the axis of the casing.

Thereafter, a casting cap was placed, and hollow sections at ends of the hollow-fiber membrane bundle were sealed with resin by centrifugal potting method (under a centrifugal force of about 80 G) to form potting layers, and both ends of the hollow-fiber membrane bundle were adhered to the inner wall of the cylindrical casing with a potting material. Urethane resin was used as the potting material. The casting cap was removed after hardening of the potting layers, and both ends were cut with a sharp blade such that central portions of the hollow-fiber membranes were opened and exposed on end surfaces of the potting layers. Thereafter, to form manifolds at the both ends of the cylindrical casing, header members were bonded thereto by ultrasonic bonding. At this time, the length of the cylindrical casing along the axis of the casing was set to 285 mm, and the inner diameter d of the treating-liquid inlet port and the treating-liquid outlet port of the cylindrical casing was set to 8 mm. The inner diameter was increased near the both ends of the casing, and the treating-liquid inlet port and the treating-liquid outlet port were disposed at the respective diameter-increasing portions. Immediately below the ports, upper baffle plates each extending from the bottom end of the diameter-increasing portion on the casing inner circumferential surface toward the front end of the casing were provided with the gap S of 3 mm from the inner openings of the respective ports. The upper baffle plates were disposed so as to cover the inner openings of the respective ports when viewed from the holes of the treating-liquid ports. The widths W1 of both the upper baffles were set to 13 mm. Further, lower baffle plates are disposed at positions where the lower baffle plates are line symmetric to the respective upper baffle plates with respect to the axis of the casing. Similarly to the upper baffle plates, the lower baffle plate extends from the bottom end of the diameter-increasing portion on the casing inner circumferential surface toward the front end of the casing. To facilitate resin molding of the cylindrical casing, the width W2 of the lower baffle plate was set to 13 mm, which is the same as the width W1 of the upper baffle plate. The gap S' from the casing inner circumferential surface was set to 3 mm to produce a blood purifier according to the present invention (Example 1). The header members were made of the same material as that of the cylindrical casing, that is, polycarbonate.

In Comparison Example, a general dialyzer was produced in the same manner as in Example 1 except that the lower baffle plate was not provided below one of the treating-liquid inlet port and the treating-liquid outlet port (Comparison Example 1). A plurality of baffle plates were disposed over the entire circumference with a gap of 3 mm from the inner opening of the treating-liquid inlet port of the cylindrical casing, and slits extending in an oblique direction with respect to the axis of the cylindrical casing were provided between the adjacent baffle plates to produce a dialyzer (Comparison Example 2).

Through comparison and evaluation of the three dialyzers, the results in Table 1 were found. The results show that the dialyzer in Example is superior to the other dialyzers in performance.

[Table 1]

| Setting Condition | Example 1 | Comparison Example 1 | Comparison Example 2 |
|---|---|---|---|
| Upper Baffle Plate Width Wi1 (mm) | 13 | 13 | Entire Circumference |
| Lower Baffle Plate Width Wi2 (mm) | 13 | 0 | |
| Performance Evaluation Item Urea Clearance (ml/min) | 194 | 190 | 189 |

The solute clearance measurement was performed according to "Dialyzer Function Classification Implementation Manual" issued on August, 1, 2009 and edited by Japan Association of Medical Devices Industries. The manual describes two types of measurement methods, and measurement conditions at the time of measurement of clearance were used as references in this experiment. The clearance was calculated by the following equation.

[Equation 1]

$$CL = \frac{QBi \times CBi - QBo \times CBo}{CBi}$$

Where, CL: clearance (ml/min)
QBi: Blood-side inlet flow rate (ml/min)
QBo: Blood-side outlet flow rate (ml/min)
CBi: Blood-side inlet solute concentration
CBo: Blood-side outlet solute concentration

INDUSTRIAL APPLICABILITY

A hollow-fiber membrane module and a cylindrical casing used in the hollow-fiber membrane module according to the present invention are applicable to hemocatharsis as described above, and are preferably particularly used as a dialyzer for artificial dialysis. However, the present invention is also applicable to a structure of a hollow-fiber membrane module used for purification of waste water, production of drinking water, and a humidification device for a fuel battery.

DESCRIPTION OF REFERENCE SIGNS

1: Header member
2: Treated-liquid inlet port
3: Potting layer
4: Opened end surface of hollow section
5: Hollow-fiber membrane bundle
6: Potting layer
7: Opened end surface of hollow section
8: Header member
9: Treated-liquid outlet port
10: Cylindrical casing
11: Treating-liquid inlet port
12: Treating-liquid outlet port
13: Upper baffle plate
14: Upper baffle plate
15: Lower baffle plate
16: Lower baffle plate
17: Inner opening of treating-liquid port of cylindrical casing
18: Inner circumference of cylindrical casing
d: Inner diameter of treating-liquid port
S: Minimum distance between casing-side end of inner opening of treating-liquid port of cylindrical casing and upper baffle plate
S': Minimum distance between casing-side end of inner opening of treating-liquid port of cylindrical casing and lower baffle plate
W1: Width of upper baffle plate
W2: Width of lower baffle plate

**Claims**

1. A hollow-fiber membrane module comprising:

a cylindrical casing having a treating-liquid inlet port and a treating-liquid outlet port;
a hollow-fiber membrane bundle mounted within the cylindrical casing while being aligned in one direction;
potting layers fixing both ends of the hollow-fiber membrane bundle to an inner wall of the cylindrical casing such that hollow sections of hollow-fiber membranes are open; and
header members surrounding opened end surfaces of the hollow-fiber membrane bundle, the header members attached to respective ends of the cylindrical casing and having a treated-liquid inlet port and a treated-liquid outlet port,
wherein the hollow-fiber membrane module further includes
an upper baffle plate provided in the cylindrical casing at a position immediately below the treating-liquid inlet port with a predetermined gap from an inner opening of the treating-liquid inlet port of the cylindrical casing, and
a lower baffle plate provided at a position at which the lower baffle plate is line symmetric to the upper baffle plate with respect to an axis of the casing, and
no baffle plate is present on an inner circumference of the cylindrical casing between the upper baffle plate and the lower baffle plate.

2. The hollow-fiber membrane module according to claim 1, wherein
the upper baffle plate covers the inner opening of the port when the port is viewed from below.

3. The hollow-fiber membrane module according to claim 1 or 2, wherein
given that an inner diameter of the treating-liquid inlet port is di, a width of the upper baffle plate is Wi1, and a width of the lower baffle plate is Wi2, $di \leq Wi1 \leq 3di$ and $di \leq Wi2 \leq 3di$ hold.

4. The hollow-fiber membrane module according to any of claims 1 to 3, wherein
the upper baffle plates and the lower baffle plates are disposed below both of the treating-liquid inlet port and the treating-liquid outlet port.

5. The hollow-fiber membrane module according to claim 4, wherein
given that an inner diameter of the treating-liquid outlet port is do, a width of the upper baffle plate is Wo1, and a width of the lower baffle plate is Wo2, $do \leq Wo1 \leq 3do$ and $do \leq Wo2 \leq 3do$ hold.

6. The hollow-fiber membrane module according to any of claims 1 to 5, which is used as a blood treatment device.

7. A cylindrical casing used in a hollow-fiber membrane module, the casing comprising a treating-liquid inlet port and a treating-liquid outlet port at a side of a body, wherein
the cylindrical casing further includes
an upper baffle plate provided in the cylindrical casing at a position immediately below the treating-liquid inlet port with a predetermined gap from an inner opening of the treating-liquid inlet port of the cylindrical casing, and
a lower baffle plate provided at a position at which the lower baffle plate is line symmetric to the upper baffle plate with respect to an axis of the casing,
the upper baffle plate covers the inner opening of the port when the port is viewed from below, and
no baffle plate is present on an inner circumference of the cylindrical casing between the upper baffle plate and the lower baffle plate.

8. The cylindrical casing according to claim 7, wherein
the upper baffle plate covers the inner opening of the port when the port is viewed from below.

9. The cylindrical casing according to claim 7 or 8, wherein
given that an inner diameter of the treating-liquid inlet port or the treating-liquid outlet port is di, a width of the upper baffle plate is Wi1, and a width of the lower baffle plate is Wi2, $di \leq Wi1 \leq 3di$ and $di \leq Wi2 \leq 3di$ hold.

10. The cylindrical casing according to any of claims 7 to 9, wherein
the upper baffle plates and the lower baffle plates are disposed below both of the treating-liquid inlet port and the treating-liquid outlet port.

**11.** The cylindrical casing according to claim 10, wherein
given that an inner diameter of the treating-liquid inlet port or the treating-liquid outlet port is do, a width of the upper baffle plate is Wo1, and a width of the lower baffle plate is Wo2, do $\leq$ Wo1 $\leq$ 3do and do $\leq$ Wo2 $\leq$ 3do hold.

**12.** The cylindrical casing according to any of claims 7 to 11, which is used in a hollow-fiber membrane module for blood treatment.

Fig. 1

Treated fluid enters

Treating fluid exits

Treating fluid enters

Treated fluid exits

1
2
4
3
16
12
14
5
10
13
15
6
7
11
8
9

Fig. 2

Fig. 3

Treating fluid enters

Fig. 4

Fig. 5

Fig. 6

Treated fluid enters

Treating fluid exits

Treating fluid enters

Treated fluid exits

Fig. 7

## INTERNATIONAL SEARCH REPORT

International application No.

PCT/JP2012/082510

### A. CLASSIFICATION OF SUBJECT MATTER
*B01D63/02*(2006.01)i, *A61M1/18*(2006.01)i

According to International Patent Classification (IPC) or to both national classification and IPC

### B. FIELDS SEARCHED

Minimum documentation searched (classification system followed by classification symbols)
B01D63/02, A61M1/18

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No. 191951/1981(Laid-open No. 95202/1983) (Kuraray Co., Ltd.), 28 June 1983 (28.06.1983), fig. 3 (Family: none) | 1-12 |
| A | JP 2005-296265 A (Nikkiso Co., Ltd.), 27 October 2005 (27.10.2005), fig. 3, 5 (Family: none) | 1-12 |

☒ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>07 February, 2013 (07.02.13) | Date of mailing of the international search report<br>19 February, 2013 (19.02.13) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2012/082510

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2004-154772 A  (Toray Industries, Inc.), 03 June 2004 (03.06.2004), fig. 2, 3 & US 2004/0074833 A1    & EP 1415703 A2 | 1-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005296265 A
- JP 2004154772 A

- JP 2001170172 A

**Non-patent literature cited in the description**

- Dialyzer Function Classification Implementation Manual. 01 August 2009